(19) Europäisches Patentamt
European Patent Office
Office européen des brevets

(11) Veröffentlichungsnummer: **0 059 400**
**B1**

(12) # EUROPÄISCHE PATENTSCHRIFT

(45) Veröffentlichungstag der Patentschrift: **13.11.85**

(51) Int. Cl.[4]: **C 07 C 125/06** // C07C119/042

(21) Anmeldenummer: **82101308.3**

(22) Anmeldetag: **20.02.82**

(54) **Verfahren zur Herstellung von Urethanen.**

(30) Priorität: **04.03.81 DE 3108067**

(43) Veröffentlichungstag der Anmeldung: **08.09.82 Patentblatt 82/36**

(45) Bekanntmachung des Hinweises auf die Patenterteilung: **13.11.85 Patentblatt 85/46**

(84) Benannte Vertragsstaaten: **BE DE FR GB IT**

(56) Entgegenhaltungen: **EP - A - 0 018 581**

(73) Patentinhaber: **BAYER AG, Konzernverwaltung RP Patentabteilung, D-5090 Leverkusen 1 Bayerwerk (DE)**

(72) Erfinder: **Fauss, Rudolf, Dr., Gerstenkamp 10, D-5000 Köln 80 (DE)**
Erfinder: **Findeisen, Kurt, Dr., In der Follmühle 10, D-5068 Odenthal (DE)**
Erfinder: **Heitkämper, Peter, Dr., Fliederweg 14, D-4047 Dormagen 11 (DE)**
Erfinder: **König, Klaus, Dr., Heymannstrasse 50, D-5090 Leverkusen (DE)**
Erfinder: **Penninger, Stefan, Dr., Pommernallee 5, D-4047 Dormagen (DE)**

**EP 0 059 400 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von N-substituierten Urethanen durch Umsetzung von primären Aminen und Alkoholen mit Allophansäureestern (Allophanaten) oder mit Allophansäureester sowie Harnstoff und/oder Carbamidsäureester (Carbaminate) enthaltenden Gemischen.

Urethane entstehen bekanntlich u. a. durch Umsetzung von organischen Isocyanaten mit Alkoholen. Diese Umsetzung ist reversibel, d. h. Urethane können thermisch in das ihnen zugrundeliegende Isocyanat und den ihnen zugrundeliegenden Alkohol gespalten werden (vgl. z. B. US-PS 2 409 712). Thermisch in Isocyanate spaltbare Urethane sind daher interessante Ausgangsmaterialien zur Herstellung dieser Isocyanate, die bislang weltweit in größerem Umfang durch Umsetzung von primären Aminen mit Phosgen hergestellt werden. Die phosgenfreie Herstellung von Urethanen und deren anschließende thermische Spaltung stellt eine interessante Alternative zu der genannten großtechnischen Isocyanatherstellung dar. Eine Methode zur phosgenfreien Herstellung von Urethanen besteht in der Umsetzung von Harnstoff mit Aminen und Alkohol (vgl. z. B. US-PS 2 409 712, US-PS 2 806 015, DE-OS 2 917 493 oder DE-OS 2 917 569).

In den DE-OS 2 917 490 und 2 917 568 ist die Urethansynthese durch Umsetzung von Aminen, O-substituierten Carbaminaten und Alkoholen beschrieben:

$$R-NH_2 + R'O\overset{\displaystyle O}{\overset{\|}{C}}NH_2 \xrightarrow[-NH_3]{R'OH} R-NHCOOR'$$

In der Europäischen Patentanmeldung 0 018 581 wird die gleichzeitige Verwendung von Harnstoff und O-substituierten Carbaminaten als Carbonylgruppenspender beschrieben.

O-substituierte Carbaminate können z. B. durch Umsetzung von Harnstoff mit Alkoholen hergestellt werden (P. Adams, F. A. Baron, Chem. Rev. 65, 569 (1965)).

Nebenprodukte dieser Reaktion sind die sich aus Harnstoff und Carbaminaten bildenden Allophansäureester. Wie aus dem zitierten Übersichtsartikel zu ersehen ist, hat es nicht an Versuchen gefehlt, durch entsprechenden Aufwand diese Nebenreaktionen stark zurückzudrängen und die Carbaminate zu reinigen. Aber selbst die von A. M. Paquin (Z. f. Naturforschung 1, 518 (1948)), sowie K. Billig und H. Krauss (DE-PS 753 127) unabhängig voneinander gefundene Verbesserung der Carbaminatherstellung aus Alkoholen und Harnstoff unter Katalyse liefert immer noch Allophanate als Nebenprodukte (s. DE-PS 753 127, Beispiel 2).

Überraschenderweise wurde nunmehr gefunden, daß auch Allophansäureester, d. h. die bislang bei der Carbaminatherstellung anfallenden, unerwünschten Nebenprodukte als Carbonylgruppen aufweisende organische Verbindungen bei der Herstellung von Urethanen aus primären Aminen, Alkoholen und Carbonylgruppen aufweisenden Verbindungen einsetzbar sind. Anstelle von reinen Allophanaten können hierbei auch deren Gemische mit Harnstoff und/oder N-unsubstituierten Carbamidsäureestern eingesetzt werden.

Dieser Befund ist überraschend, da in einer sehr ausführlichen Untersuchung über die Umsetzung zwischen Aminen und Allophanaten (F. B. Dains, E. Wertheim, Am. Soc. 42, 2304 (1920)) z. B. unter den Folgeprodukten der Reaktion von Allophansäureethylester mit Anilin $\omega$-Phenylallophansäureethylester und $\omega,\omega'$-Diphenylbiuret, aber kein N-Phenyl-O-ethylurethan gefunden wurde.

Gegenstand der vorliegenden Erfindung ist somit ein Verfahren zur Herstellung von N-substituierten Urethanen der Formel

$$R^1-\left[NH-\overset{\displaystyle O}{\overset{\|}{C}}-O-R^2\right]_n$$

in welcher

$R^1$ für einen aliphatischen Kohlenwasserstoffrest mit 3 bis 18 Kohlenstoffatomen, einen cycloaliphatischen Kohlenwasserstoffrest mit 6 bis 15 Kohlenstoffatomen oder einen gegebenenfalls Methyl-, Methoxy- oder Chlor-substituierten und gegebenenfalls Methylenbrücken aufweisenden aromatischen Kohlenwasserstoffrest mit 6 bis 15 Kohlenstoffatomen steht,

$R^2$ für einen $C_1-C_4$-Alkoxy- oder $C_1-C_4$-Alkoxy-$C_2-C_4$-alkoxy-substituierten oder unsubstituierten aliphatischen Kohlenwasserstoffrest mit 1 bis 18, insbesondere einen gesättigten unsubstituierten aliphatischen Kohlenwasserstoffrest mit 1 bis 4 Kohlenstoffatomen oder für einen Cyclohexyl- oder 2-Phenylethyl-Rest steht und

n 1 oder 2 bedeutet,

durch Umsetzung von

a) primären Aminen der Formel

$R^1(NH_2)_n$

und

b) Alkoholen der Formel

$R^2-OH$

mit

c) Carbonylgruppen aufweisenden organischen Verbindungen im Temperaturbereich von 130 bis 350°C, dadurch gekennzeichnet, daß man als Carbonylgruppen aufweisende organische Verbindungen c) Allophansäureester der Formel

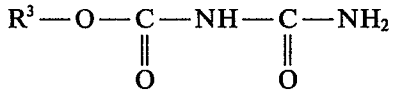

oder mindestens 20 Äquivalentprozent derartiger Allophansäureester aufweisende Gemische aus c 1) Allophansäureestern, c 2) Harnstoff und/oder c 3) Carbamidsäureestern der Formel

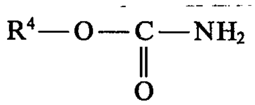

verwendet, wobei
$R^3$ für einen Rest steht, der gleich oder verschieden von $R^2$ ist, und der bezüglich seiner Bedeutung der Definition von $R^2$ entspricht, und
$R^4$ gleich oder verschieden von $R^2$ und $R^3$ ist und bezüglich seiner Bedeutung im übrigen der Definition von $R^2$ entspricht.

Ausgangsmaterialien für das erfindungsgemäße Verfahren sind

a) primäre Amine der Formel

$R^1(NH_2)_n$,

in welcher $R^1$ und n die bereits genannte Bedeutung haben.

Beispiele geeigneter Amine sind Methylamin, Ethylamin, Propylamin, Isopropylamin, Butylamin, iso-Butylamin, tert.-Butylamin, Hexylamin, Dodecylamin, 2-Ethylhexylamin, Tetradecylamin, Hexadecylamin, Octadecylamin, Allylamin, 1,4-Diaminobutan, 1,6-Diaminohexan, 2,5-Dimethyl-2,5-hexandiamin, Trimethylhexamethylendiamin, Cyclohexylamin, Trimethylcyclohexylamin, 2-Norbornylmethylamin, Anilin, o,m,p-Chloranilin, 2,3-, 2,4-, 2,5-, 2,6-Dichloranilin, 3,4-Dichloranilin, p,o-Nitroanilin, m,o,p-Tolylamin, 3-Chlor-4-methylanilin, Benzylamin, Phenyl-cyclohexylamin, Naphthylamin, 1,4-Diaminocyclohexan, 2,4-, 2,6-Diamino-1-methylcyclohexan, 5-Amino-1-aminomethyl-1,3,3-trimethylcyclohexan, 4,4'-Diaminodicyclohexylmethan, 4,4'-Diamino-3,3'-dimethyl-dicyclohexylamin, 1,3-Diaminobenzol, 1,4-Diaminobenzol, 2-Chlor-1,4-diaminobenzol, 2,4-Diaminotoluol, 2,6-Diaminotoluol (und Gemische mit 2,4-) 2-(N-Ethyl-amino)-4-aminotoluol, 1,3-Diamino-2-methylbenzol, 1,3-Bisaminomethylbenzol, 1,3-Diamino-2,6-(4,6)-diethyl-4-methylbenzol, 1,3-Diamino-2,4,6-triisopropylbenzol, 1,5-Diaminonaphthalin, 2,7-Diaminonaphthalin, Benzidin, 3,3'-Dichlorbenzidin, 4,4'-Diaminodiphenylmethan (und Rohwaren), 3,3'-Dichlor-4,4'-diamino-diphenylmethan, 2,2-Bis-(4-aminophenyl)-propan, 1,1'-Bis-(4-aminophenyl)-cyclohexan, 1,1'-Bis-(4-amino-3-methylphenyl)-cyclohexan, p-Methoxyanilin, p-Ethoxyanilin, 2,4-Diaminodiphenylether, 3,5-Diamino-2-methyl-diphenylmethan, 3,5-Diamino-4-methyldiphenylmethan (und Gemische), 3,5-Diamino-4-methyl-dicyclohexylmethan, 3,5-Diamino-2-methyldicyclohexylmethan (und Gemische).

Besonders bevorzugte Amine sind Propylamin, Isopropylamin, n-Butylamin, sec.-Butylamin, tert.-Butylamin, Stearylamin, Hexamethylendiamin, Cyclohexylamin, 3,3,5-Trimethyl-5-aminomethylcyclohexylamin, 4,4'-Diaminodicyclohexylmethan, Anilin, p-Chloranilin, 3,4-Dichloranilin, m-Tolylamin, p-Methoxyanilin, 3,4-Dichloranilin, m-Tolylamin, p-Methoxyanilin, 2,4-Diaminotoluol, 2,6-Diaminotoluol, 4,4'-Diaminodiphenylmethan, 2,4'-Diaminodiphenylmethan oder technische Gemische der genannten Diaminotoluole bzw. Diaminodiphenylmethane.

Weitere Ausgangsmaterialien sind b) Alkohole der allgemeinen Formel

$R^2-OH$,

in welcher
$R^2$ die oben angegebene Bedeutung hat.

Beispiele geeigneter Alkohole sind Methanol, Ethanol, Propanol, iso-Propanol, Butanol, iso-Butanol, Pentanol, iso-Pentanol, Hexanol, iso-Hexanol, Heptanol, iso-Heptanol, Octanol, iso-Octanol, Nonanol, iso-Nonanol, Decanol, iso-Decanol, Dodecanol, 2-Ethylhexanol, $\beta$-Chlorethanol, 2-Ethylbutanol, Hexadecanol, Octadecanol, Fettalkohol-Gemische, 2-Methoxyethanol, 2-Ethoxyethanol, 2-Propoxyethanol, 2-Butoxyethanol, 2-(2-Butoxyethoxy)-ethanol, Cyclohexanol, 2-Phenylethanol, 1-Phenylethanol.

Bevorzugte Alkohole sind Methanol, Ethanol, n-Propanol, iso-Propanol, n-Butanol, iso-Butanol, Cyclohexanol, n-Hexanol, 2-Ethylhexanol, $\beta$-Phenylethanol, Glykolmonomethylether, Glykolmonobutylether oder Diglykolmonomethylether. Besonders bevorzugte Alkohole sind die erstgenannten $C_1-C_4$-Alkanole.

Weitere Ausgangsmaterialien für das erfindungsgemäße Verfahren sind c 1) Allophansäureester der allgemeinen Formel

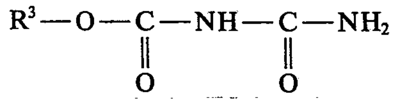

in welcher

$R^3$ für einen Rest steht, der gleich oder verschieden von $R^2$ ist, und der bezüglich seiner Bedeutung der Definition von $R^2$ entspricht.

Anstelle dieser Alkansäureester c 1) können beim erfindungsgemäßen Verfahren auch Gemische derartiger Allophansäureester c 1) mit c 2) Harnstoff und/oder c 3) Carbamidsäureestern der Formel

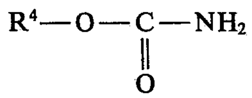

eingesetzt werden, wobei

$R^4$ gleich oder verschieden von $R^2$ und $R^3$ ist und bezüglich seiner Bedeutung im übrigen der Definition von $R^2$ entspricht.

Falls als Reaktionskomponente c) derartige Gemische eingesetzt werden, enthalten diese mindestens 20 Äquivalentprozent und besonders bevorzugt mindestens 40 Äquivalentprozent der genannten Allophansäureester. Das Verhältnis der Komponenten c 2) und c 3) in derartigen Gemischen kann innerhalb beliebiger Grenzen variieren. Unter »Äquivalentprozent« sind hierbei »CO-Äquivalentprozente« zu verstehen. So entspricht beispielsweise 1 Mol Harnstoff oder 1 Mol Carbamidsäureester c 3) einem »CO-Grammäquivalent« und 1 Mol Allophansäureester c 1) 2 »CO-Grammäquivalenten«.

Beispiele von für das erfindungsgemäße Verfahren besonders gut geeigneten Allophanaten bzw. Carbaminaten sind Methyl-, Ethyl-, n-Propyl-, iso-Propyl-, n-Butyl-, iso-Butyl-, Cyclohexyl- oder n-Hexyl-carbaminat bzw. -allophanat. Die entsprechenden $C_1-C_4$-Alkyl-Derivate sind besonders bevorzugt.

Wie bei der Definition der Reste $R^3$ und $R^4$ bereits erwähnt sind diese Reste gleich oder verschieden von dem Rest $R^2$ des jeweils eingesetzten Alkohols. Insbesondere bei Verwendung von Alkoholen $R^2-OH$, die einen höheren Siedepunkt als die Alkohole $R^3-OH$ bzw. $R^4-OH$ aufweisen, ist es durchaus möglich, solche Ausgangsmaterialien einzusetzen, für welche die Bedeutung der genannten Reste unterschiedlich ist, da in einem solchen Falle die leichter flüchtigen Alkohole $R^3-OH$ bzw. $R^4-OH$ vom schwerer flüchtigen Alkohol $R^2-OH$ verdrängt würden und destillativ entfernt werden können.

Bei der Durchführung des erfindungsgemäßen Verfahrens kommen die Amine a) im allgemeinen in 0,5—4fach, vorzugsweise 0,8—1,5fach und insbesondere 0,9—1,1fach stöchiometrischen Mengen und die Alkohole b) im allgemeinen in 1—10fach, vorzugsweise 1,1—4fach und insbesondere 1,2—2fach stöchiometrischen Mengen, jeweils bezogen auf die Summe der Carbonylgruppen der Komponenten C) bzw. c 1), c 2) und c 3) zum Einsatz.

Das erfindungsgemäße Verfahren kann in Gegenwart von Katalysatoren durchgeführt werden, wobei erhöhte Raumzeit-Ausbeuten erreichbar sind. Überraschenderweise wurde gefunden, daß beliebige Verbindungen, die einen katalytischen Einfluß auf die Veresterungsreaktion von Carbonsäuren mit Alkoholen ausüben auch eine beschleunigende Wirkung auf die erfindungsgemäße Umsetzung haben. Für das erfindungsgemäße Verfahren besonders gut geeignete Katalysatoren sind

(i) unter den Reaktionsbedingungen inerte anorganische oder organische Basen, (ii) Lewis-Säuren und (iii) Salze bzw. Komplexverbindungen, insbesondere Chelate von Übergangsmetallen.

Beispiele geeigneter Katalysatoren der Gruppe (i) sind tert.-Amine wie Tri-n-propylamin, Triethylamin, Triisopentylamin, Diethylbenzylamin, N,N-Dimethyl-benzylamin, Hexahydrodimethylanilin, N-Ethylpiperazin, Diethyl-(2-methoxypropyl)-amin, 2-(Diethylammoethyl)-phenylether, Oxethylmorpholin, N-(2-Diethylaminoethyl)-benzamid, N-(2-Diethylaminoethyl)-propionamid, 1,4-Diaza-(2,2,2)-bicyclooctan, N,N-Dimethyl-4-aminopyridin, 1-Azabicycloheptane, 1-Azabicyclooctane, gesättigte polyheterocyclische Amine, wie 3-Methylconidin, 1-Azabicyclo-(3,2,1)-octan, Pyrrolizidine und Chinuclidine, anorganische Basen wie Berylliumhydroxid und Natrium-, Kalium-, Lithium-, Magnesium-, Barium- oder Calciumhydroxid, basische Alkalisalze wie Natriumcarbonat, Natriumsulfid, Kaliumcarbonat oder Trinatriumphosphat sowie Alkalisalze von Fettsäuren oder Sulfonsäuren.

Geeignete Katalysatoren (ii) sind beispielsweise Lewis-Säuren wie Eisen-II-chlorid, Eisen-III-chlorid, Zinkchlorid, Zinn-II-chlorid, Zinn-IV-chlorid, Aluminiumchlorid, Zinkcyanid, Thalliumtrichlorid, Bortrifluorid oder Bortrifluoridetherat.

Geeignete Katalysatoren der Gruppe (iii) sind beispielsweise Salze von Übergangsmetallen, soweit sie nicht bereits in die Gruppe (ii) fallen, sowie Komplexverbindungen insbesondere Chelate dieser Metalle wie Kobalt-, Mangan- oder Beinaphthenate, Eisenoleate oder -carbonyle, Acetylacetonate von Eisen, Nickel, Kobalt, Zink, Blei, Aluminium, Mangan, Magnesium, Molybdän, Titan, Torium, Zirkon oder Vanadium, Bis-(dibenzoylmethan)-kupfer, Bis-(ethylacetoacetat)-kupfer, -eisen, Koordinationsverbindungen von Titan, Zirkon, Hafnium, Thorium und Mangan mit $\beta$-Diketonen, $\beta$-Ketoestern und $\beta$-Hydroxyaldehyden, Dibutylzinnlaurat, Dibutylzinndiacetat, Di-(2-ethylhexyl)-zinnoxid, Dioctylzinnoxid, Zinnsalze von $C_1-C_{20}$-Carbonsäuren wie Zinn-(II)-naphthenat, -hexoat, -calmitat, -stearat oder -di-methylvalertat, Acetate, Chloride, Sulfate oder Octoate des zweiwertigen Kupfers, des Zinks oder des zweiartigen Bleis.

Besonders gut geeignete Katalysatoren sind beispielsweise Zinkchlorid, Zinkacetat, Zinkoctoat, Zinkoxid, Zinkcyanid, Zinn-II-chlorid, Zinn-IV-chlorid, Dibutylzinndilaurat, Kobalttriacetat, Kobalttrichlorid, Kobalttrioctoat, Kupfer(II)-sulfat, Bleiacetat oder Bleichlorid.

Die Menge des jeweils eingesetzten Katalysators liegt im allgemeinen zwischen 1 ppm und 20 Gew.-%, bevorzugt zwischen 100 ppm und 5 Gew.-%, bezogen auf die Summe aller Ausgangsmaterialien. Man wird in der Praxis selbstverständlich bestrebt bleiben, die Konzentration der Katalysatoren möglichst gering zu halten. Die optimale Konzentration hängt von der Art der Ausgangsmaterialien und der Aktivität des jeweiligen Katalysators ab und kann in einem einfachen Versuch bestimmt werden.

Die Durchführung des erfindungsgemäßen Verfahrens kann sowohl unter Druck als auch drucklos erfolgen. Die Anwendung von Druck beispielsweise im Bereich von 1 bis 80 bar ist oft dann sinnvoll, wenn die Reaktionstemperatur oberhalb dem Siedepunkt einer oder mehrerer der Ausgangsmaterialien liegt. Das erfindungsgemäße Verfahren wird im allgemeinen im Temperaturbereich von 120° C bis 350° C, vorzugsweise 130 bis 300° C und insbesondere 140° C bis 250° C durchgeführt.

Das erfindungsgemäße Verfahren kann in Gegenwart oder auch in Abwesenheit von Lösungsmitteln durchgeführt werden. Geeignete Lösungsmittel sind beispielsweise unter den Verfahrensbedingungen inerte Lösungsmittel mit einem zwischen 100° C und 280° C, vorzugsweise zwischen 150° C und 250° C liegenden Siedepunkt. Beispiele geeigneter Lösungsmittel sind n-Nonan, n-Butylcyclohexan, Decahydronaphthalin, n-Undecan, n-Dodecan, n-Hexylcyclohexan, Dipenten, 1-Dodecen, Isopropylbenzol, 1,3-Diethylbenzol, Inden, n-Butylbenzol, Tetralin, Chlorbenzol, 4-Chlortoluol, 1,2-Dichlorbenzol, 2,4-Dichlortoluol, 1,2,4-Trichlorbenzol, 2-Chlor-4-isopropyl-1-methylbenzol, Anisol, Cyclohexylethylether, Diethylenglykoldimethylether, Benzylmethylether, 4-Methoxytoluol, Parachloranisol, Di-n-hexylether, Phenyl-n-propylketon, Benzophenon, Acetophenon, Formamid, N,N-Dimethylformamid, N,N-Diethylformamid, N-Methylformamid, Dimethylacetamid, N-Methylpyrrolidon, Caprolactam, Phenol, substituierte Phenole, Sulfolan, Hexamethylphosphorsäuretriamid, Dimethylsulfoxid, Ethylenglykolmonomethyletheracetat, Di-n-propylcarbonat, Cyclohexylacetat, Diisobutylcarbonat, 2-Ethylpyridin, N,N-Dimethyl-2-methylanilin, N,N-Dimethylanilin, N-Methyl-N-ethylanilin, N,N-Dimethyl-2-chloranilin, N,N-Diethylanilin, Chinolin, Nitrocyclohexan, Nitrobenzol, 2-Nitrotoluol, 2,4-Dimethyl-1-nitrobenzol, Acetonitril, N-Capronitril, Benzonitril, Tolunitril und Phenylacetonitril, besonders bevorzugt sind polare Lösungsmittel und deren Gemische.

Die Mitverwendung derartiger Lösungsmittel ist oft, beispielsweise bei Verwendung eines hohen Überschusses an Alkohol (Komponente b)) überflüssig. Insbesondere erübrigt sich die Verwendung derartiger Hilfslösungsmittel bei der Herstellung von Monourethanen (n = 1), d. h. bei der ausschließlichen Verwendung von Monoaminen als Komponente a).

Zur Durchführung des erfindungsgemäßen Verfahrens werden die Reaktionspartner im allgemeinen während eines Zeitraumes von 1 bis 20 Stunden auf die Reaktionstemperatur erhitzt, wobei darauf geachtet werden muß, daß der sich bildende Ammoniak entweichen kann. Die Aufarbeitung erfolgt in an sich bekannter Weise, insbesondere destillativ, wobei die Verfahrensprodukte meistens als letzte Fraktion oder als Destillationsrückstand anfallen. Vor dieser destillativen Aufarbeitung der Reaktionsansätze können erforderlichenfalls unlösliche Bestandteile (z. B. unlösliche Katalysatoren) abfiltriert werden. Die Verfahrensprodukte können dann ohne weitere Reindarstellung der erfindungsgemäßen

Verwendung zugeführt, d. h. in an sich bekannter Weise thermisch in das ihnen zugrundeliegende Isocyanat und den ihnen zugrundeliegenden Alkohol gespalten werden.

Die in den nachfolgenden Beispielen erwähnten Prozentangaben beziehen sich auf Gewichtsprozente.

## Beispiel 1

186 g (2 Mol) Anilin, 240 g (2,4 Mol) Cyclohexanol, 132 g (1 Mol) Ethylallophanat und 0,8 g Zn-octoat werden zügig unter Ammoniakabspaltung auf 200° C geheizt. Durch Zugabe von weiterem Cyclohexanol wird die Rückflußtemperatur von 200°C aufrechterhalten, wobei entstandenes Ethanol laufend abdestilliert wurde. Nach insgesamt 11 Stunden haben sich 91% d. Th. O-Cyclohexyl-N-phenylurethan gebildet (Untersuchung mittels Hochdruckflüssigkeitschromatographie).

## Beispiel 2

Es wird verfahren wie bei Beispiel 1, jedoch wird kein Katalysator zugesetzt. Nach 14 Stunden haben sich nur 85% d. Th. gewünschtes Urethan gebildet.

## Beispiel 3

204,6 g (2,2 Mol) Anilin, 60 g (1 Mol) Harnstoff, 66 g (0,5 Mol) Ethylallophanat, 366 g (3 Mol) $\beta$-Phenylethanol und 1 g Zn-octoat wurden 10 h auf 200° C erhitzt. Ethanol wurde abdestilliert. Mit Hilfe der Hochdruckflüssigkeitschromatographie wurde die Ausbeute an N-Phenyl-O-$\beta$-phenylethylurethan zu 89% d. Th. bestimmt.

## Beispiel 4

122 g (1 Mol) 2,4-Diaminotoluol, 30 g Harnstoff (0,5 Mol), 33 g (0,25 Mol) Ethylallophanat, 178 g (2 Mol) Carbamidsäureethylester, 255 g (2,5 Mol) n-Hexanol wurden mit 2,5 g Zn-octoat versetzt und auf 205° C erhitzt. Nach 9 Stunden wurde mit Hilfe der Hochdruckflüssigkeitschromatographie die Ausbeute an 2,2-Bis-(n-hexoxy-carbonylamino)-toluol zu 80% d. Th. bestimmt.

## Beispiel 5

204,6 g (2,2 Mol) Anilin, 160,2 g (1,8 Mol) Carbamidsäureethylester, 13,2 g (0,1 Mol) Ethylallophanat und 255 g (2,5 Mol) Cyclohexanol wurden mit 1 g Zn-octoat zum Rückfluß erhitzt. Entstehendes Ethanol wurde laufend abdestilliert. Nach 10 Stunden Reaktionszeit wurde die Ausbeute an O-Cyclohexyl-N-phenylurethan mit Hilfe der Hochdruckflüssigkeitschromatographie zu 91% d. Th. bestimmt.

## Beispiel 6

324 g (2 Mol) 3,4-Dichloranilin, 60 g (1 Mol) Harnstoff, 35,6 g (0,4 Mol) Carbamidsäureethylester, 66 g (0,5 Mol) Ethylallophanat, 300 g (3 Mol) Cyclohexanol und 1,5 g Zinkoctoat wurden am Rückfluß erhitzt. Nach 2 Stunden war die Sumpftemperatur auf 205° C gestiegen und wurde durch langsame Zugabe von weiteren 70 ml Cyclohexanol weitere 8 Stunden gehalten. Mit Hilfe der Hochdruckflüssigkeitschromatographie wurde die Ausbeute an O-Cyclohexyl-N-3,4-dichlorphenylurethan zu 92% der Theorie bestimmt.

**Patentansprüche**

1. Verfahren zur Herstellung von N-substituierten Urethanen der Formel

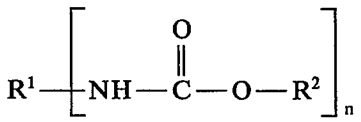

in welcher

$R^1$ für einen aliphatischen Kohlenwasserstoffrest mit 3 bis 18 Kohlenstoffatomen, einen cycloaliphatischen Kohlenwasserstoffrest mit 6 bis 15 Kohlenstoffatomen oder einen gegebenenfalls Methyl-, Methoxy- oder Chlor-substituierten und gegebenenfalls Methylenbrücken aufweisenden aromatischen Kohlenwasserstoffrest mit 6 bis 15 Kohlenstoffatomen steht,
$R^2$ für einen $C_1-C_4$-Alkoxy- oder $C_1-C_4$-Alkoxy-$C_2-C_4$-alkoxy-substituierten oder unsubstituierten aliphatischen Kohlenwasserstoffrest mit 1 bis 18 Kohlenstoffatomen oder für einen Cyclohexyl- oder 2-Phenylethyl-Rest steht und
n 1 oder 2 bedeutet,

durch Umsetzung von

a) primären Aminen der Formel

$R^1(NH_2)_n$

und

b) Alkoholen der Formel

$R^2-OH$

mit

c) Carbonylgruppen aufweisenden organischen Verbindungen im Temperaturbereich von 130 bis 350°C, dadurch gekennzeichnet, daß man als Carbonylgruppen aufweisende organische Verbindungen c) Allophansäureester der Formel

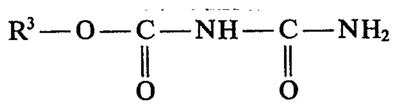

oder mindestens 20 Äquivalentprozent derartiger Allophansäureester aufweisende Gemische aus c 1) Allophansäureestern, c 2) Harnstoff und/oder c 3) Carbamidsäureestern der Formel

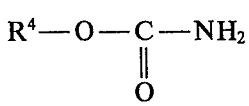

verwendet, wobei
$R^3$ für einen Rest steht, der gleich oder verschieden vor $R^2$ ist, und der bezüglich seiner Bedeutung der Definition von $R^2$ entspricht, und
$R^4$ gleich oder verschieden von $R^2$ und $R^3$ ist und bezüglich seiner Bedeutung im übrigen der Definition von $R^2$ entspricht.

2. Verfahren gemäß Anspruch 1, dadurch gekennzeichnet, daß man die Umsetzung in Gegenwart von Veresterungskatalysatoren für Carbonsäuren als Katalysatoren durchführt.

3. Verfahren gemäß Anspruch 1 und 2, dadurch gekennzeichnet, daß man die Umsetzung in polaren Lösungsmitteln durchführt.

4. Verfahren gemäß Anspruch 3, dadurch gekennzeichnet, daß man als Lösungsmittel überschüssige Mengen des als Reaktionspartner zum Einsatz gelangenden Alkohols verwendet.

## Claims

1. Process for the preparation of N-substituted urethanes of the formula

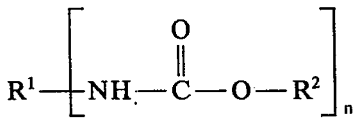

in which

$R^1$ represents an aliphatic hydrocarbon radical with 3 to 18 carbon atoms, a cycloaliphatic hydrocarbon radical with 6 to 15 carbon atoms or an optionally methyl-, methoxy- or chlorine-substituted aromatic hydrocarbon radical which has 6 to 15 carbon atoms and optionally contains methylene bridges,

$R^2$ represents a $C_1-C_4$-alkoxy- or $C_1-C_4$-alkoxy-$C_2-C_4$-alkoxy-substituted or unsubstituted aliphatic hydrocarbon radical with 1 to 18 carbon atoms, or a cyclohexyl or 2-phenylethyl radical and

n denotes 1 or 2,

by reacting

a) primary amines of the formula

$R^1(NH_2)_n$

and

b) alcohols of the formula

$R^2-OH$

with

c) organic compounds containing carbonyl groups, at a temperature in the range from 130 to 350° C, characterised in that allophanic acid esters of the formula

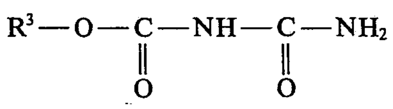

or mixtures, containing at least 20 equivalent percent of such allophanic acid esters, of c 1) allophanic acid esters, c 2) urea and/or c 3) carbamic acid esters of the formula

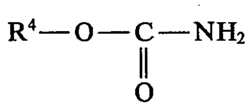

wherein
$R^3$ represents a radical which is identical to or different from $R^2$ and which corresponds in its meaning to the definition of $R^2$, and
$R^4$ ist identical to or different from $R^2$ and $R^3$ and otherwise corresponds in its meaning to the definition of $R^2$, are used as the organic compounds c) containing carbonyl groups.

2. Process according to Claim 1, characterised in that the reaction is carried out in the presence of esterification catalysts for carboxylic acids, as the catalysts.

3. Process according to Claim 1 and 2, characterised in that the reaction is carried out in polar solvents.

4. Process according to Claim 3, characterised in that excess quantities of the alcohol used as a co-reactant are used as the solvent.

**Revendications**

1. Procédé de fabrication d'uréthanes N-substitués de formule:

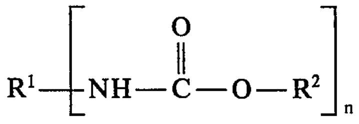

dans laquelle

$R^1$ représente un radical hydrocarboné aliphatique ayant 3 à 18 atomes de carbone, un radical hydrocarboné cycloaliphatique ayant 6 à 15 atomes de carbone, ou un radical hydrocarboné aromatique éventuellement méthyl-, méthoxy- ou chlorosubstitué et présentant éventuellement des ponts méthylène, ayant 6 à 15 atomes de carbone,
$R^2$ représente un radical hydrocarboné aliphatique alcoxy($C_1-C_4$)- ou alcoxy ($C_1-C_4$)-alcoxy-($C_2-C_4$)-substitué, ou non substitué, ayant 1 à 18 atomes de carbone, ou un radical cyclohexyle ou 2-phényléthyle, et
n signifie 1 ou 2,

par réaction

a) d'amines primaires de formule

$R^1(NH_2)_n$

et

b) d'alcools de formule

$R^2-OH$

avec

c) des composés organiques présentant des groupes carbonyle dans l'intervalle de température de 130 à 350° C, caractérisé en ce qu'on utilise comme composés organiques c) présentant des groupes carbonyle des esters allophaniques de formule:

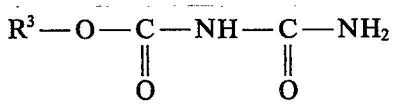

ou des mélanges présentant au moins 20% d'équivalent de ces esters allophaniques et se composant c 1) d'esters allophaniques, c 2) d'urée et/ou c 3) d'esters carbamiques de formule

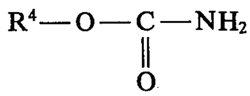

où
$R^3$ représente un radical qui est identique ou différent de $R^2$ et qui, quant à sa signification, correspond à la définition de $R^2$, et où
$R^4$ est identique ou différent de $R^2$ et de $R^3$ et correspond, quant à sa signification, pour le restant à la définition de $R^2$.

2. Procédé selon la revendication 1, caractérisé en ce qu'on exécute la réaction en présence de catalyseurs d'estérification pour acides carboxyliques.

3. Procédé selon les revendications 1 et 2, caractérisé en ce qu'on exécute la réaction dans des solvants polaires.

4. Procédé selon la revendication 3, caractérisé en ce qu'on utilise en tant que solvant les quantités excédentaires de l'alcool employé comme partenaire de réaction.